# EUROPÄISCHE PATENTSCHRIFT

(11) **EP 2 281 600 B1**
(45) Veröffentlichungstag und Bekanntmachung des Hinweises auf die Patenterteilung: **23.11.2011**
(21) Anmeldenummer: 10007276.8
(22) Anmeldetag: 14.07.2010
(51) Int. Cl.: A61N 1/05

(54) **Vorrichtung zur Defibrillation eines Herzens**
Device for defibrillating a heart
Dispositif de défibrillation d'un coeur

(30) Priorität: 06.08.2009 DE 102009036357
(43) Veröffentlichungstag der Anmeldung: 09.02.2011
(73) Patentinhaber: Osypka, Peter, 79618 Rheinfelden-Herten (DE)
(72) Erfinder: Osypka, Peter, 79618 Rheinfelden-Herten (DE)
(74) Vertreter: Maucher, Wolfgang

(56) Entgegenhaltungen:
- WO-A2-03/041791
- DE-B3-102005 016 364
- US-A- 5 483 022
- VERMA A ET AL: "Intravascular pacemaker and defibrillator lead extraction: A state-of-the-art review" HEART RHYTHM, ELSEVIER, US LNKD- DOI:10.1016/J.HRTHM.2004.09.020, Bd. 1, Nr. 6, 1. Dezember 2004 (2004-12-01), Seiten 739-745, XP004680983 ISSN: 1547-5271

## Beschreibung

Die Erfindung betrifft eine Vorrichtung zur Defibrillation eines Herzens mit einem implantierbaren Herzschrittmacher und Defibrillator, mit zumindest einer Defibrillationselektrode und zugehöriger Gegenelektrode dafür sowie mit wenigstens einer ebenfalls implantierbaren Stimulationselektrode, wobei die Defibrillationselektrode in Gebrauchsstellung mit der Stimulationselektrode derart verbunden ist, dass der Pol der Defibrillationselektrode an der Außenseite der Stimulationselektrode angeordnet ist.

Eine vergleichbare Vorrichtung ist aus WO 03/041791 A2 bekannt und für solche Patienten geeignet, bei denen trotz einer ständig möglichen Stimulation Herzkammerflimmern auftreten kann, welchem dann sofort mit dem Defibrillator und der integrierten Defibrillationselektrode entgegengewirkt werden muss.

Da die Defibrillationselektrode in der Regel als Pol eine Wendel aufweist, die an der Außenseite der Stimulationselektrode verläuft, und weil diese häufig aus Platin, einem relativ spröden Metall besteht, welches zwar eine gute elektrische Leitfähigkeit hat, aber durch die beschriebene Anordnung der Dynamik der Herzbewegungen ausgesetzt ist, kann es zu Brüchen dieser Defibrillationselektrode oder ihrer Wendel kommen, auch wenn diese aus anderem Werkstoff besteht, so dass deren wirksame Länge verkürzt wird und für eine Defibrillation und den dafür erforderlichen Schock möglicherweise nicht mehr ausreicht.

Zwar könnte man die implantierte Elektrodenanordnung insgesamt explantieren und durch eine reparierte und neue ersetzen, jedoch bedeutet gerade für Patienten, die eine derartige Vorrichtung und Elektrodenanordnung benötigen, eine solche Operation ein in aller Regel zu hohes Risiko.

Es besteht deshalb die Aufgabe, eine Vorrichtung der eingangs definierten Art zu schaffen, bei welcher der durch einen Bruch der Defibrillationselektrode oder ihres Pols entstandener Schaden ohne Explantation der Stimulationselektrode behoben werden kann.

Zur Lösung dieser scheinbar widersprüchlichen Aufgabe ist die eingangs genannte Vorrichtung dadurch gekennzeichnet, dass die Defibrillationselektrode relativ zu der sie tragenden Stimulationselektrode verschiebbar und relativ zu der implantierten Stimulationselektrode aus ihrer Gebrauchslage zurückziehbar und auswechselbar ist.

Durch diese erfindungsgemäße Trennung der Defibrillationselektrode von der Stimulationselektrode bei dennoch räumlicher Zuordnung kann also eine eventuell schadhafte oder gebrochene Defibrillationselektrode dadurch entfernt werden, dass ein sie oder ihren Pol tragender Schlauch oder dergleichen Halterung zurückgezogen und mit einer neuen Defibrillationselektrode versehen oder durch eine komplette neue Defibrillationselektrode mit eigenem Schlauch oder eigener Halterung ersetzt wird.

Es ist also zweckmäßig und vorteilhaft, wenn der Pol der Defibrillationselektrode als elektrisch leitende Wendel ausgebildet ist, die an einer relativ zu der Stimulationselektrode verschiebbaren Halterung angeordnet ist, welche mit der die Defibrillationselektrode oder deren Pol bildenden Wendel zurückziehbar und auswechselbar ist.

Dabei kann die Halterung für die Defibrillationselektrode, wie schon erwähnt, ein innenliegender Schlauch sein, der auf der Stimulationselektrode in Gebrauchsstellung verschiebbar angeordnet ist und gegebenenfalls lösbar festgelegt werden kann. Es kann aber auch ein außenliegender Schlauch sein, der eine gegebenenfalls gebrochene Defibrillationselektrode umfasst und bei einer Entnahme zusammenhält.

Eine vorteilhafte Ausführungsform der erfindungsgemäßen Vorrichtung kann vorsehen, dass die Stimulationselektrode in ihrem Verlauf nahe dem Herzschrittmacher oder ihrem Stecker dafür eine lösbare Kupplung oder Trennstelle aufweist oder lösbar mit dem Herzschrittmacher verbunden ist und dass die Halterung oder der Schlauch mit der Defibrillationselektrode und deren Anschlüssen als Steckteil mit einer Abzweigung für die Anschlüsse versehen ist, welches Steckteil nach dem Lösen der Stimulationselektrode von dem Herzschrittmacher oder an der Kupplungsstelle oder an der Trennstelle auf den zum Herzen führenden Teil der Stimulationselektrode aufsteckbar und darauf verschiebbar oder davon abziehbar ist. Somit können beide Elektroden konzentrisch und relativ zueinander verschiebbar angeordnet sein, aber ihre eigenen Anschlüsse für den Herzschrittmacher und Defibrillator haben.

Günstig ist es dabei, wenn die die Defibrillationselektrode oder deren Pol aufweisende Halterung - insbesondere ein Schlauch - mit der Abzweigung oder einer parallelen Haltevorrichtung und einer Befestigungsstelle an der Stimulationselektrode verschiebbar und festlegbar ausgebildet ist. Die Abzweigung weist dann beispielsweise den oder die entsprechenden Anschlüsse für den Defibrillator auf.

Die Halterung oder der Schlauch können benachbart zu der Abzweigung eine Klemme aufweisen, die in gelöster Position relativ zu der Stimulationselektrode verschiebbar und in Klemmstellung daran festgelegt ist. Es kann also mit Hilfe einer konzentrisch zur Stimulationselektrode vorgesehenen Klemme die relative Lage von Stimulationselektrode und Defibrillationselektrode in Gebrauchsstellung fixiert werden, wobei die Klemme in Fortsetzung der Defibrillationselektrode parallel zu deren Anschluss oder Anschlüssen angeordnet sein kann, so dass sie auf der Stimulationselektrode in Offenstellung verschiebbar ist und mit dieser verklemmt werden kann, wenn die gesamte Vorrichtung ihre Gebrauchslage hat. Ist die Klemme gelöst worden, kann sie und die mit ihr verbundene Defibrillationselektrode an der Trennstelle der Stimulationselektrode von dieser abgezogen werden. Ist anschließend die reparierte oder erneuerte Defibrillationselektrode wieder an der Trennstelle auf die Stimulationselektrode aufgeschoben und mit ihr verbunden worden, kann die Stimulationselektrode an ihrer Trennstelle auch wieder geschlossen oder verbunden werden, wonach die gesamte Vorrichtung erneut funktionstüchtig ist.

Die Klemme zum Fixieren der Defibrillationselektrode auf der Stimulationselektrode kann eine Hülse mit einer in axialer Richtung durchgehenden Innenhöhlung zur Aufnahme der Stimulationselektrode aufweisen, wobei die Hülse einen in axialer Richtung gegen eine Rückstellkraft elastisch verformbaren Einsatz oder dehnbaren und/oder elastischen Schlauch enthält, der gleichzeitig die Innenlängshöhlung zur Aufnahme der Elektrode bildet oder umfasst, wobei die Hülse in Längsrichtung entlang einer an ihrem Umfang umlaufenden Trennstelle in wenigstens zwei in axialer Richtung relativ zueinander bewegbare Teile unterteilt ist, wobei die Trennstelle gegenüber einer Durchmesserebene abwechselnd axial nach entgegengesetzten Seiten abweichend umläuft oder profiliert oder verzahnt ist, so dass die sich stirnseitig berührenden Ränder der Teile der Hülse in Drehrichtung formschlüssig verbunden sind, wobei ferner die Teile der Hülse in axialer Richtung entgegen der Rückstellkraft des sie zusammenhaltenden elastischen Einsatzes oder Schlauches soweit auseinanderziehbar sind, dass die gegenseitige Profilierung gelöst und unter Tordierung oder Verwringung des elastischen Einsatzes oder Schlauches relativ zueinander verdrehbar und in verdrehter Position wiederum in Berührung miteinander gegen Drehbewegungen festlegbar sind.

Eine derartige Klemme ist sehr einfach bedienbar, weil der Benutzer nur ihre beiden Teile relativ zueinander bewegen muss, wozu auch die axiale Bewegung eines der beiden Teile genügt, wonach eine Relativverdrehung zur Verengung der Innenhöhlung führt, so dass nach dem Zusammenführen der beiden Teile in ihre Gebrauchsstellung eine Klemmwirkung auftritt. Die Klemme entspricht also weitgehend einer in anderem Zusammenhang bereits vorbekannten Klemme gemäß DE 10 2005 016 364 B3 und hat auch die darin beschriebenen Vorteile.

Die den Defibrillationspol bildende Wendel kann in Längsrichtung in wenigstens zwei Abschnitte unterteilt sein, deren Abstand so gewählt ist, dass der eine Abschnitt zum Anordnen im Ventrikel und der andere Abschnitt zum Anordnen im Vorhof des Herzens vorgesehen sind. Entsprechend effektiv ist die koaxial zur der Stimulationselektrode angeordnete Defibrillationselektrode.

Eine Weiterbildung kann dabei vorsehen, dass der Abstand der den Defibrillationspol bildenden Abschnitte veränderbar ist. Dies ermöglicht eine Anpassung an unterschiedliche anatomische Verhältnisse.

Zweckmäßig kann es dabei sein, wenn zur Veränderung des Abstandes der den Defibrillationspol bildenden Abschnitte diese jeweils auf in konzentrisch zueinander angeordneten Halterungen oder Schlauchstücken angeordnet sind, deren längere Halterung oder deren längeres Schlauchstück den Abschnitt der Defibrillationselektrode trägt, der in Gebrauchsstellung im Ventrikel angeordnet ist, und dass der relativ dazu verstellbare oder verschiebbare andere Abschnitt der Wendel der Defibrillationselektrode auf einer kürzeren, an der Außenseite der erstgenannten Halterung oder des Schlauchstücks angeordneten Halterung oder einem Schlauchstück angebracht ist. Auf diese Weise ist die zur Anpassung an die jeweilige Anatomie eines Herzens für die Defibrillation optimale Einstellung des Abstands der beiden Abschnitte und Pole auf einfache Weise möglich.

Dabei ist es vorteilhaft, wenn die beiden Schlauchstücke in Gebrauchsstellung fest oder durch eine Klemme verbunden und beim Auswechseln der Defibrillationselektrode gemeinsam relativ zu der Stimulationselektrode verschiebbar oder zurückziehbar sind. Ferner können die Abschnitte miteinander über eine weitere Klemme verbunden sein, die für eine Relativverschiebung der beiden Abschnitte lösbar ist.

Denkbar ist aber auch, dass eine einzige Klemme zum Befestigen der Defibrillationselektrode insgesamt auf der Stimulationselektrode vorgesehen ist, mit der gleichzeitig die beiden Abschnitte der Defibrillationselektrode in Gebrauchsstellung aneinander festlegbar sind.

Eine Ausgestaltung der Erfindung insbesondere zur Verminderung der erforderlichen Energie beim Defibrillieren kann darin bestehen, dass der Polbereich der Defibrillationselektrode, insbesondere die den oder die Pole bildende Wendel der Defibrillationselektrode - gegebenenfalls an ihren beiden Abschnitten - mit einem einzelne Öffnungen oder Fenster aufweisenden Isolier- oder Silikonschlauch überzogen ist. An den Öffnungen kann jeweils der Strom fließen, wird aber dann auf diese Öffnungen oder Fenster beschränkt, so dass entsprechend weniger Strom abgegeben werden muss. Gleichzeitig wird die Wendel der Defibrillationselektrode gegen ein Einwachsen von Bindegewebe geschützt.

Die Öffnungen oder Fenster des Isolierschlauches können Schlitze oder Lochungen oder wenigstens eine über zumindest einen Teil des Umfangs des Isolierschlauches verlaufende Unterbrechung sein.

Eine weitere Ausgestaltung zur Erhöhung der Effektivität kann vorsehen, dass im Bereich einer Öffnung, eines Fensters oder einer Unterbrechung des Isolierschlauches, an der die Defibrillationselektrode und deren Pol bildenden Wendel wenigstens eine Platinhülse angeordnet ist. Diese hat eine sehr gute Leitfähigkeit und ist gleichzeitig mit dem umgebenden Gewebe biokompatibel.

Die erfindungsgemäße Vorrichtung kann dahingehend ausgestaltet sein, dass die Defibrillationselektrode und die von ihr aufgenommene oder umschlossene Stimulationselektrode in zusammengesteckter Lage implantierbar sind, wobei die Defibrillationselektrode aufgrund ihrer relativen axialen Verschiebbarkeit gegenüber der Stimulationselektrode relativ zu dieser so weit verschoben sein kann, dass sie das distale Verankerungsende oder eine dieses bildende Schraubwendel der Stimulationselektrode - womit diese in Gebrauchsstellung im Herzgewebe fixiert ist - in sich aufnimmt und umschließt, und dass am Ende des Einführungsvorganges die Defibrillationselektrode relativ zu der Stimulationselektrode in ihre Gebrauchsstellung zurückziehbar und justierbar und/oder festlegbar ist. Diese Festlegung kann beispielsweise mit der schon beschriebenen und erläuterten Klemme erfolgen.

Die Verschiebbarkeit der Defibrillationselektrode wird also dazu ausgenutzt, die Implantation zu verbessern und zu vereinfachen insbesondere dann, wenn die Stimulationselektrode in vorteilhafter Weise an ihrem distalen Ende eine Schraubwendel für ihre Fixierung aufweist, die während des Implantationsvorganges ohne eine solchen Schutz durch die Defibrillationselektrode mit der Gefäßinnenwand in Berührung kommen und diese verletzen könnte, sofern sie nicht ihrerseits relativ zu der Stimulationselektrode zurückziehbar gestaltet ist. Die Stimulationselektrode mit Schraubwendel kann also entsprechend einfach und ohne einen Mechanismus zum Verstellen der Schraubwendel gestaltet sein, weil die Schraubwendel während der Implantation von der erfindungsgemäß verschiebbaren Defibrillationselektrode umschlossen werden kann.

Vor allem bei Kombination einzelner oder mehrerer der vorbeschriebenen Merkmale und Maßnahmen ergibt sich eine aus Stimulations- und Defibrillationselektrode bestehende Vorrichtung, bei welcher die Defibrillationselektrode relativ zu der Stimulationselektrode verschiebbar und somit aufgrund einer möglichen Trennung der Stimulationselektrode von dem Herzschrittmacher oder in ihrem Verlauf vollständig von der Stimulationselektrode abgezogen werden kann, um sie gegen eine neue Defibrillationselektrode auszutauschen. In zweckmäßiger Weise ist dabei die Stimulationselektrode im Bereich ihrer Verbindung mit dem Herzschrittmacher von diesem lösbar so gestaltet, dass ein nach dem Lösen freies Ende dieser Stimulationselektrode gebildet ist, dessen Querschnitt das Aufstecken und Aufschieben der ein durchgehendes Lumen aufweisenden Defibrillationselektrode ermöglicht. Die Trennstelle der Stimulationselektrode ist also zweckmäßiger Weise so gestaltet, dass deren Stecker während des Aufsteckens oder des Lösens der Defibrillationselektrode von dieser gelöst ist.

Nachstehend sind Ausführungsbeispiele der Erfindung anhand der Zeichnung näher beschrieben. Es zeigt in zum Teil schematisierter Darstellung:
- Figur 1: eine implantierte erfindungsgemäße Vorrichtung zur Defibrillation mit einem in Gebrauchsstellung ange- ordneten kombinierten Herzschrittmacher und De- fibrillator, wobei die Defibrillationselektrode konzentrisch außenseitig auf der Stimulationselek- trode angeordnet und ihr Anschluss an einer Abzwei- gung parallel zu dem Anschluss der Stimulations- elektrode verschiebbar vorgesehen ist, wobei der einzige als Wendel ausgebildete Pol der Defibrilla- tionselektrode so lang ist, dass er im Vorhof und im Ventrikel gleichzeitig wirksam ist,
- Figur 2: eine der Figur 1 entsprechende Darstellung, wobei der Pol der Defibrillationselektrode unterteilt ist und die beiden Abschnitte einen Abstand derart auf- weisen, dass der eine Abschnitt im Vorhof und der andere Abschnitt im Ventrikel angeordnet sind,
- Figur 3: in vergrößertem Maßstab die Stimulationselektrode und die erfindungsgemäß konzentrisch zu dieser an- geordnete Defibrillationselektrode, die eine Hal- terung und eine Abzweigung für ihren Anschluss hat, wobei die Halterung von der Stimulationselektrode durchsetzt ist und die Stimulationselektrode eine Kupplung für ihren Anschluss an dem Herzschrittma- cher hat, wobei eine Klemme zwischen diesem An- schluss und der Abzweigung vorgesehen ist, womit die Stimulationselektrode an dem durch die Abzwei- gung führenden Teil der Stimulationselektrode fest- legbar ist,
- Figur 4: die Anordnung gemäß Figur 3 nach dem Lösen der Trennstelle der Stimulationselektrode, wodurch die Stimulationselektrode über das im Bereich der Tren- nung freie Ende der Stimulationselektrode zurück- ziehbar und wieder aufsteckbar ist,
- Figur 5: die freie Stimulationselektrode und die von ihr ge- trennte, abgezogene oder aufsteckbare Stimulations- elektrode mit der an ihr befindlichen Klemme,
- Figur 6: eine abgewandelte Ausführungsform, bei welcher der Pol der Defibrillationselektrode unterteilt ist und für jeden Abschnitt im Bereich der Abzweigung ein eigener Anschluss vorgesehen ist gemäß der Ausfüh- rungsform nach Figur 2,
- Figur 7: die Trennung der Stimulationselektrode von ihrem Anschluss, wobei die mit zwei Polen versehene De- fibrillationselektrode noch auf die Stimulations- elektrode aufgesteckt ist,

- Figur 8: eine Darstellung gemäß Figur 7 und analog Figur 5 nach dem Abziehen oder vor dem Aufstecken der De- fibrillationselektrode auf die Stimulationselek- trode,
- Figur 9: ein Ausführungsbeispiel, bei welchem die Defibrillationselektrode von einem Isolierschlauch überzogen ist, der Öffnungen für seine Pole hat,
- Figur 10: die Defibrillationselektrode gemäß Figur 9 nach dem Lösen von der Stimulationselektrode oder vor ihrem Aufstecken darauf sowie
- Figur 11 und Figur 12: abgewandelte Ausführungsbeispiele von Defibrilla- tionselektroden, deren Pole mit einem Isolier- schlauch überzogen sind, welcher unterschiedliche Öffnungen oder Lochungen hat.

Bei der nachstehenden Beschreibung unterschiedlicher Ausführungsbeispiele erhalten hinsichtlich ihrer Funktion übereinstimmende Teile auch bei abgewandelter Gestaltung über einstimmende Bezugszahlen.

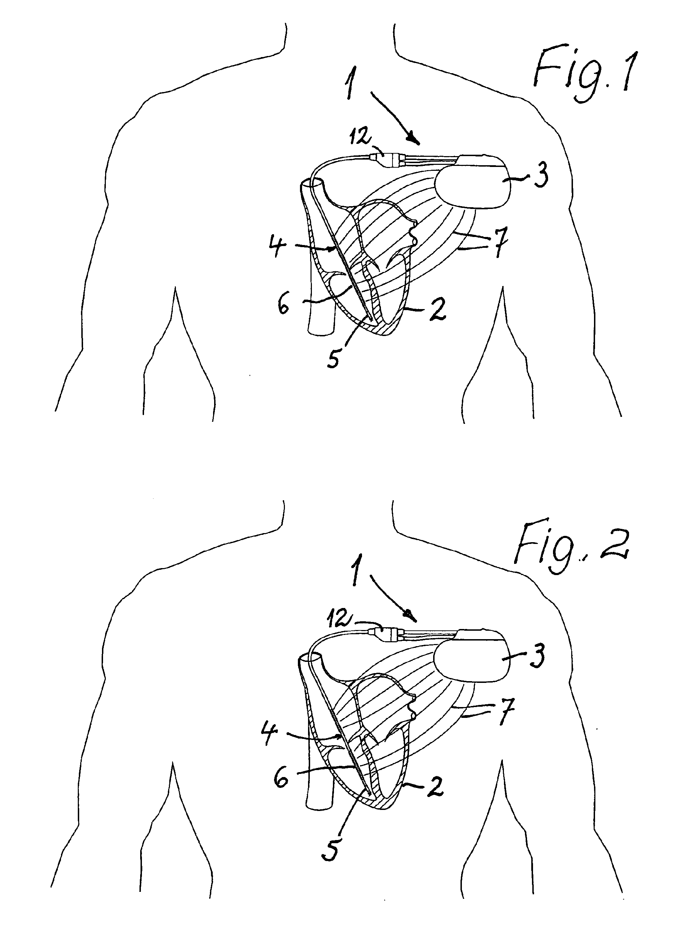

Eine im Ganzen mit 1 bezeichnete Vorrichtung dient zur Defibrillation eines Herzens 2 und umfasst einen implantierbaren kombinierten Herzschrittmacher und Defibrillator 3, im Folgenden auch nur "Herzschrittmacher 3" oder "Defibrillator 3" genannt, eine Defibrillationselektrode 4 mit zugehöriger Gegenelektrode, die durch das Herzschrittmachergehäuse gebildet ist, sowie eine ebenfalls implantierbare, in Figur 1 und 2 in Gebrauchsstellung dargestellte Stimulationselektrode 5, die gleichzeitig als Sensing-Elektrode wirksam ist.

Gemäß den Figuren 1, 2, 3, 4, 6, 7 und 9 ist die Defibrillationselektrode 4 in Gebrauchsstellung mit der Stimulationselektrode 5 in noch zu beschreibender Weise derart verbunden, dass der Pol 6 der Defibrillationselektrode 4 an der Außenseite der Stimulationselektrode 5 angeordnet ist.

Auf diese Weise kann eine sehr wirksame Defibrillation mit einer günstigen Feldstärkeverteilung durchgeführt werden, die in den Figuren 1 und 2 durch Feldlinien 7 angedeutet ist.

Bei allen Ausführungsbeispielen ist vorgesehen, dass die Defibrillationselektrode 4 relativ zu der Stimulationselektrode 5, diese umschließend, verschiebbar und relativ zu der implantierten Stimulationselektrode 5 aus ihrer Gebrauchsstellung zurückziehbar und in noch zu beschreibender Weise auswechselbar ist. Somit ist es möglich, bei Fehlfunktionen der Defibrillationselektrode 4, die beispielsweise durch einen Bruch auftreten können, diese ohne eine aufwendige Operation entfernen zu können, weil die Stimulationselektrode 5 implantiert bleiben kann. Mit einem relativ kleinen Eingriff ist es möglich, die Defibrillationselektrode 4 von der Stimulationselektrode 5 abzuziehen und durch eine neue Defibrillationselektrode 4 zu ersetzen.

In den Ausführungsbeispielen ist der schon erwähnte Pol 6 der Defibrillationselektrode 4 als elektrisch leitende Wendel ausgebildet, was in den Zeichnungen angedeutet ist. Diese den Pol 6 bildende Wendel ist dabei in nicht näher dargestellter Weise an einer relativ zu der Stimulationselektrode 5 verschiebbaren Halterung angeordnet, die in zweckmäßiger Weise ein Schlauch 8 ist und mit der Defibrillationselektrode 4 oder deren Pol bildenden Wendel zurückziehbar und auswechselbar ist. Der als Halterung dienende Schlauch 8 ist also in Gebrauchsstellung auf der Stimulationselektrode 5 verschiebbar und in noch zu beschreibender Weise festlegbar angeordnet.

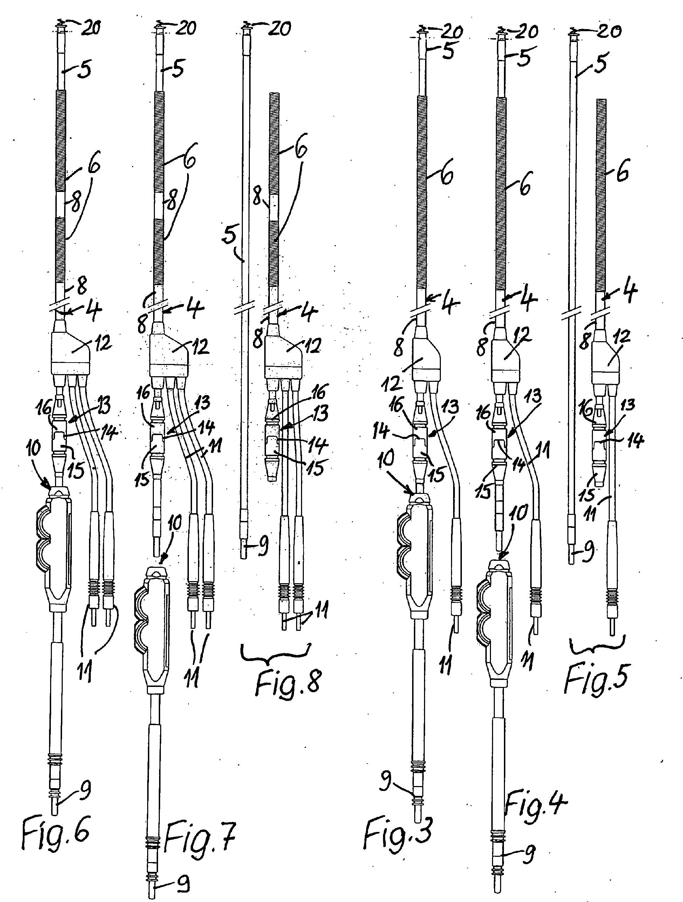

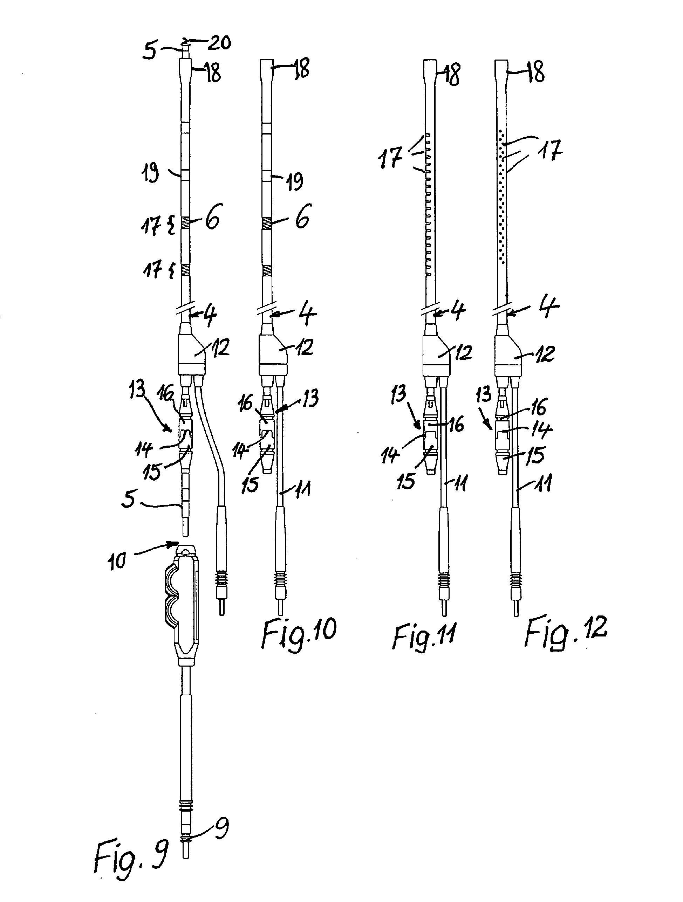

Beim Vergleich der Figuren 3 und 4 oder 6 und 7 sowie anhand von Figur 9 erkennt man, dass die Stimulationselektrode 5 in ihrem Verlauf nahe dem Herzschrittmacher 3 auf der dem Herzschrittmacher 3 abgewandten Seite ihres darin einführbaren Steckers 9 eine lösbare Kupplung oder Trennstelle 10 aufweist, die in Figur 3 und 6 geschlossen oder verbunden und in Figur 4 und 7 geöffnet oder getrennt ist.

Ferner erkennt man in diesen und in weiteren Figuren, dass die Halterung oder der Schlauch 8 mit der Defibrillationselektrode 4 und deren Anschluss oder Anschlüssen 11 für den Defibrillator 3 als Steckteil mit einer Abzweigung 12 für diese Anschlüsse 11 versehen ist. Der oder die Anschlüsse 11 sind also innerhalb dieser Abzweigung 12 mit dem oder den Polen 6 der Defibrillationselektrode 4 elektrisch verbunden.

Dieses aus Schlauch 8 und Abzweigung 12 bestehende Steckteil kann nach dem Lösen der Stimulationselektrode 5 von dem Herzschrittmacher 3 beziehungsweise an der Trennstelle 10 auf den zum Herzen 2 führenden Teil der Stimulationselektrode 5 aufgesteckt und darauf verschoben werden oder bei einem Austausch kann dieser Steckteil von der Stimulationselektrode 5 über ihr abgetrenntes Ende abgezogen werden.

Anstelle einer Kupplung mit Trennstelle 10 könnte auch der Stecker 9 unmittelbar mit der Stimulationselektrode 5 lösbar verbunden sein, um das Aufstecken oder Abziehen der Defibrillationselektrode ohne Kollision mit dem Stecker 9 zu erlauben.

Die die Defibrillationselektrode 4 oder deren Pol 6 aufweisende Halterung, also der Schlauch 8, ist mit der Abzweigung 12 und einer noch zu beschreibenden Befestigungsstelle an der Stimulationselektrode 5 verschiebbar und festlegbar ausgebildet. Dazu ist im Ausführungsbeispiel vorgesehen, dass die Halterung oder der Schlauch 8 benachbart zu der Abzweigung 12 auf deren distaler Seite eine Klemme 13 aufweist, die von der Stimulationselektrode 5 in Gebrauchsstellung durchsetzt ist und die in gelöster Position relativ zu der Stimulationselektrode 5 verschiebbar und in Klemmstellung daran festgelegt ist.

Nach dem Trennen der Stimulationselektrode 4 im Bereich ihrer Trennstelle 10 kann also die Defibrillationselektrode 4 mit ihrer Abzweigung 12 und der Klemme 13 auf die Stimulationselektrode 5 aufgeschoben werden, wie umgekehrt nach einer solchen Trennung die Defibrillationselektrode 4 mit ihrer Abzweigung 12 und ihrer Klemme 13 von der Stimulationselektrode 5 abgezogen werden kann. Figur 5 und 8 zeigt jeweils nebeneinander die ab ihrer Trennstelle 10 entsprechend schlanke Stimulationselektrode 5, auf welche die daneben abgebildete Defibrillationselektrode 4 mit ihrer Abzweigung 12 und der Klemme 13 aufgeschoben werden kann. In gleicher Weise sind diese Figuren die Darstellung nach einer entsprechenden Trennung der beiden Elektroden.

Die Klemme 13 ist im Ausführungsbeispiel derart ausgebildet, wie eine Klemme für einen anderen Anwendungszweck in DE 10 2005 016 364 B3 beschrieben ist. Sie weist zum Fixieren der Defibrillationselektrode 4 auf der Stimulationselektrode 5 eine in der genannten Patentschrift näher dargestellte Hülse mit einer in axialer Richtung durchgehende Innenhöhlung zur Aufnahme der Stimulationselektrode 5 auf, wobei diese Hülse der Klemme 13 einen in axialer Richtung gegen eine Rückstellkraft elastisch verformbaren Einsatz oder dehnbaren und/oder elastischen Schlauch enthält, der gleichzeitig die Innenlängshöhlung zur Aufnahme der Stimulationselektrode 5 bildet und umfasst. Die Hülse ist in Längsrichtung entlang einer an ihrem Umfang umlaufenden Trennstelle 14 in zwei in axialer Richtung relativ zueinander bewegbare Teile 15 und 16 unterteilt, wobei die Trennstelle 14 gegenüber einer senkrecht zur Zeichenebene verlaufenden Durchmesserebene abwechselnd axial nach entgegengesetzten Seiten abweichend umläuft, also eine von einer ringförmigen Kontur abweichende Profilierung hat oder verzahnt ist, so dass die sich stirnseitig berührenden Ränder der Teile 15 und 16 in Drehrichtung formschlüssig verbunden sind. Wie in DE 10 2005 016 364 B3 erläutert, sind die Teile 15 und 16 in axialer Richtung entgegen der Rückstellkraft des sie zusammenhaltenden elastischen Einsatzes oder Schlauches soweit auseinanderziehbar, dass die gegenseitige Profilierung gelöst und die beiden Teile 15 und 16 unter Tordierung oder Verwringung und damit Verengung des elastischen Einsatzes oder Schlauches relativ zueinander verdrehbar und in verdrehter Position wiederum in Berührung miteinander bringbar und dadurch gegen Drehbewegungen festlegbar und mit der Stimulationselektrode kraftschlüssig verbindbar sind. Beispielsweise kann der Teil 15 relativ zu dem Teil 16 von diesem und von der Abzweigung 12 zurückgezogen, teilweise oder mehrfach verdreht und wieder in Gebrauchsstellung gebracht werden, nachdem die Defibrillationselektrode 4 mit der Abzweigung 12 und der Klemme 13 auf eine Stimulationselektrode 5 aufgesteckt wurde, die dadurch festgeklemmt wird.

Während in den Ausführungsbeispielen gemäß Figur 1 und 3 bis 5 die den Defibrillationspol 6 bildende Wendel durchgängig ist und gemäß Figur 1 sowohl im Vorhof als auch im Ventrikel des Herzens 2 wirksam sein kann, ist in den Ausführungsbeispielen gemäß Figur 2, 6 bis 8, 9 und 10 vorgesehen, dass diese den Defibrillationspol 6 bildende Wendel in Längsrichtung in wenigstens zwei Abschnitte unterteilt ist, deren Abstand gemäß Figur 2 so gewählt ist, dass der eine Abschnitt zum Anordnen im Ventrikel und der andere Abschnitt zum Anordnen im Vorhof des Herzens 2 vorgesehen und geeignet sind. Dabei ist für jeden dieser Abschnitte an der Abzweigung 12 ein entsprechender Anschluss 11 vorgesehen. Somit ist es möglich, diese Abschnitte jeweils mit einem Defibrillationsstrom zu beaufschlagen.

Ferner kann dieser Abstand der den Defibrillationspol 6 bildenden Abschnitte veränderbar sein, wobei für eine solche Veränderung der den oder die Defibrillationspole 6 bildenden Abschnitte jeweils auf in konzentrisch zueinander angeordneten Halterungen oder Schlauchstücken angeordnet sein können, deren längere Halterung oder deren längeres Schlauchstück den Abschnitt der Defibrillationselektrode 4 trägt, der in Gebrauchsstellung im Ventrikel angeordnet ist. Der relativ dazu verstellbare oder verschiebbare, für den Vorhof vorgesehene andere Abschnitt der Wendel der Defibrillationselektrode 4 kann dann auf einer kürzeren, an der Außenseite der erstgenannten Halterung oder des Schlauchstücks angeordneten Halterung oder einem Schlauchstück angebracht sein, so dass dieses relativ zu dem innenliegenden Schlauchstück verschoben werden kann.

Die beiden Schlauchstücke können in Gebrauchsstellung fest oder durch eine Klemme, beispielsweise auch die Klemme 13 verbunden und beim Auswechseln der Defibrillationselektrode 4 gemeinsam relativ zu der Stimulationselektrode 5 verschiebbar oder zurückziehbar sein.

In den Figuren 9 bis 12 sind abgewandelte Ausführungsbeispiele dargestellt, bei welchen der Polbereich der Defibrillationselektrode 4 und dabei die den oder die Pole 6 bildende Wendel mit einem einzelne Öffnungen 17 oder Fenster aufweisenden Isolier- oder Silikonschlauch 18 überzogen ist.

Figur 11 und 12 zeigt Ausführungsbeispiele, bei welchen die Öffnungen 17 oder Fenster des Isolierschlauches 18 Schlitze (Figur 11) oder Lochungen (Figur 12) sind. In Figur 9 und 10 ist dargestellt, dass die Öffnungen 17 am Umfang des Isolierschlauches 18 verlaufende Unterbrechungen sein können, die zweckmäßigerweise nicht über den gesamten Umfang verlaufen, so dass sie auch in Zugrichtung Kräfte übertragen können.

In Figur 9 und 10 ist noch angedeutet, dass im Bereich einer Öffnung 17 oder eines Fensters oder einer Unterbrechung des Isolierschlauches 18 an der Defibrillationselektrode als Pol 6 wenigstens eine Platinhülse 19 angeordnet sein kann, um die Reizübertragung zu verbessern.

Bei den in Figur 1 oder 2 dargestellten Vorrichtungen 1 sind also die Defibrillationselektrode 4 und die von dieser im Inneren konzentrisch aufgenommene Stimulationselektrode 5 in zusammengesteckter Lage implantierbar. Dabei ist die Defibrillationselektrode 4 zunächst relativ zu der Stimulationselektrode 5 in nicht näher dargestellter Weise so weit über deren distales Ende hinaus verschoben, dass sie das Verankerungsende und eine dieses bildende Schraubwendel 20 der Stimulationselektrode 5 in sich aufnimmt und umschließt. Betrachtet man beispielsweise Figur 9 ist leicht einsehbar, dass gegenüber der dort dargestellten Gebrauchsstellung die Defibrillationselektrode 4 so weit verschoben werden und sein kann, dass die Schraubwendel 20 am distalen Ende der Stimulationselektrode 5 von der Defibrillationselektrode 4 umschlossen wird. Am Ende eines derartigen Implantationsvorganges kann dann die Defibrillationselektrode 4 relativ zu der Stimulationselektrode 5 in ihre Gebrauchsstellung gemäß Figur 3, 6 oder 9 zurückgezogen, justiert und/oder insbesondere mit Hilfe der Klemme 13 festgelegt werden. Somit erhält die relativ zu der Stimulationselektrode 5 verschiebbare Defibrillationselektrode 4 eine Zusatzfunktion bei der ersten Implantation der gesamten Vorrichtung 1, indem sie die Schraubwendel 20 umschließt, so dass diese keine Verletzungen an der Innenseite eines Gefäßes hervorrufen kann.

Bei der Vorrichtung 1 zur Defibrillation eines Herzens 2 mit einem implantierbaren Herzschrittmacher und Defibrillator 3 ist die Defibrillationselektrode 4 auf der Stimulationselektrode 5, diese umschließend angeordnet. Um im Falle eines bei Gebrauch auftretenden Defekts an der Defibrillationselektrode 4 diese ohne aufwendige Operation entnehmen zu können, ist sie relativ zu der Stimulationselektrode 5 verschiebbar und relativ zu der implantierten Stimulationselektrode 5 aus ihrer Gebrauchslage zurückziehbar und auswechselbar, wofür die Stimulationselektrode 5 von dem Herzschrittmacher 3 oder ihrem an diesem vorgesehenen Stecker 9 lösbar oder trennbar ist.

## Patentansprüche

1. Vorrichtung (1) zur Defibrillation eines Herzens (2) mit einem implantierbaren Herzschrittmacher und Defibrillator (3), mit zumindest einer Defibrillationselektrode (4) und zugehöriger Gegenelektrode dafür sowie mit wenigstens einer ebenfalls implantierbaren Stimulationselektrode (5), wobei die Defibrillationselektrode (4) in Gebrauchsstellung mit der Stimulationselektrode (5) derart verbunden ist, dass der Pol (6) der Defibrillationselektrode (4) an der Außenseite der Stimulationselektrode (5) angeordnet ist, **dadurch gekennzeichnet, dass** die Defibrillationselektrode relativ zu der Stimulationselektrode (5) verschiebbar und relativ zu der implantierten Stimulationselektrode (5) aus ihrer Gebrauchslage zurückziehbar und auswechselbar ist.

2. Vorrichtung nach Anspruch 1, **dadurch gekennzeichnet, dass** der Pol (6) der Defibrillationselektrode (4) als elektrisch leitende Wendel ausgebildet ist, die an einer relativ zu der Stimulationselektrode (5) verschiebbaren Halterung angeordnet ist, welche mit der die Defibrillationselektrode (4) oder deren Pol (6) bildenden Wendel zurückziehbar und auswechselbar ist.

3. Vorrichtung nach Anspruch 1 oder 2, **dadurch gekennzeichnet, dass** die Halterung für die Defibrillationselektrode ein innenliegender Schlauch (8) ist, der auf der Stimulationselektrode (5) in Gebrauchsstellung verschiebbar angeordnet ist.

4. Vorrichtung nach einem der Ansprüche 1 bis 3, **dadurch gekennzeichnet, dass** die Stimulationselektrode (5) in ihrem Verlauf nahe dem Herzschrittmacher (3) oder ihrem Stecker (9) eine lösbare Kupplung oder Trennstelle (10) aufweist oder lösbar mit dem Herzschrittmacher (3) verbunden ist und dass die Halterung oder der Schlauch (8) mit der Defibrillationselektrode (4) und deren Anschluss oder Anschlüssen (11) als Steckteil mit einer Abzweigung (12) für die Anschlüsse (11) versehen ist, welches Steckteil nach dem Lösen der Stimulationselektrode (5) von dem Herzschrittmacher (3) oder an der Trennstelle (10) auf den zum Herzen (2) führenden Teil der Stimulationselektrode (5) aufsteckbar und darauf verschiebbar oder davon abziehbar ist.

5. Vorrichtung nach einem der Ansprüche 1 bis 4, **dadurch gekennzeichnet, dass** die die Defibrillationselektrode (4) oder deren Pol (6) aufweisende Halterung mit der Abzweigung (12) oder parallelen Haltevorrichtung und einer Befestigungsstelle an der Stimulationselektrode (5) verschiebbar und festlegbar ausgebildet ist.

6. Vorrichtung nach einem der Ansprüche 1 bis 5, **dadurch gekennzeichnet, dass** die Halterung oder der Schlauch (8) benachbart zu der Abzweigung (12) eine Klemme (13) aufweist, die in gelöster Position relativ zu der Stimulationselektrode (5) verschiebbar und in Klemmstellung daran festgelegt ist.

7. Vorrichtung nach einem der Ansprüche 1 bis 6, **dadurch gekennzeichnet, dass** die Klemme (13) zum Fixieren der Defibrillationselektrode (4) auf der Stimulationselektrode (5) eine Hülse mit einer in axialer Richtung durchgehenden Innenhöhlung zur Aufnahme der Stimulationselektrode (5) aufweist, wobei die Hülse einen in axialer Richtung gegen eine Rückstellkraft elastisch verformbaren Einsatz oder dehnbaren und/oder elastischen Schlauch enthält, der gleichzeitig die Innenlängshöhlung zur Aufnahme der Stimulationselektrode (5) bildet oder umfasst, und dass die Hülse in Längsrichtung entlang einer an ihrem Umfang umlaufenden Trennstelle (14) in wenigstens zwei in axialer Richtung relativ zueinander bewegbare Teile (15, 16) unterteilt ist, wobei die Trennstelle (14) gegenüber einer Durchmesserebene abwechselnd axial nach entgegengesetzten Seiten abweichend umläuft oder profiliert oder verzahnt ist, so dass die sich stirnseitig berührenden Ränder der Teile (15, 16) der Hülse in Drehrichtung formschlüssig verbunden sind, und dass die Teile (15, 16) in axialer Richtung entgegen der Rückstellkraft des sie zusammenhaltenden elastischen Einsatzes oder Schlauches soweit auseinanderziehbar sind, dass die gegenseitige Profilierung gelöst und die beiden Teile (15, 16) unter Tordierung oder Verwringung des elastischen Einsatzes oder Schlauches relativ zueinander verdrehbar und in verdrehter Position wiederum in Berührung miteinander gegen Drehbewegungen festlegbar sind.

8. Vorrichtung nach einem der Ansprüche 1 bis 7, **dadurch gekennzeichnet, dass** die den Defibrillationspol (6) bildende Wendel in Längsrichtung in wenigstens zwei Abschnitte unterteilt ist, deren Abstand so gewählt ist, dass der eine Abschnitt zum Anordnen im Ventrikel und der andere Abschnitt zum Anordnung im Vorhof des Herzens (2) vorgesehen sind.

9. Vorrichtung nach einem der Ansprüche 1 bis 8, **dadurch gekennzeichnet, dass** der Abstand der den Defibrillationspol (6) bildenden Abschnitte veränderbar ist.

10. Vorrichtung nach einem der Ansprüche 1 bis 9, **dadurch gekennzeichnet, dass** zur Veränderung des Abstands der den Defibrillationspol (6) bildenden Abschnitte diese jeweils auf in konzentrisch zueinander angeordneten Halterungen oder Schlauchstücken angeordnet sind, deren längere Halterung oder deren längeres Schlauchstück den Abschnitt der Defibrillationselektrode (4) trägt, der in Gebrauchsstellung im Ventrikel angeordnet ist, und dass der relativ dazu verstellbare oder verschiebbare andere Abschnitt der Wendel der Defibrillationselektrode (4) auf einer kürzeren, an der Außenseite der erstgenannten Halterung oder des Schlauchstücks angeordneten Halterung oder einem Schlauchstück angebracht ist.

11. Vorrichtung nach einem der Ansprüche 1 bis 10, **dadurch gekennzeichnet, dass** die beiden Schlauchstücke in Gebrauchsstellung fest oder durch eine Klemme (13) verbunden und beim Auswechseln der Defibrillationselektrode gemeinsam relativ zu der Stimulationselektrode (5) verschiebbar oder zurückziehbar sind.

12. Vorrichtung nach einem der Ansprüche 1 bis 11, **dadurch gekennzeichnet, dass** der Polbereich der Defibrillationselektrode (4), insbesondere die den oder die Pole (6) bildende Wendel der Defibrillationselektrode mit einem einzelne Öffnungen (17) oder Fenster aufweisenden Isolier- oder Silikonschlauch (18) überzogen ist.

13. Vorrichtung nach einem der Ansprüche 1 bis 12, **dadurch gekennzeichnet, dass** die Öffnungen (17) oder Fenster des Isolierschlauchs (18) Schlitze oder Lochungen oder wenigstens eine über zumindest einen Teil des Umfangs des Isolierschlauches (18) verlaufende Unterbrechung sind.

14. Vorrichtung nach einem der Ansprüche 1 bis 13, **dadurch gekennzeichnet, dass** im Bereich einer Öffnung, eines Fensters oder einer Unterbrechung des Isolierschlauches (18) an der die Defibrillationselektrode bildenden Wendel wenigstens eine Platinhülse (19) angeordnet ist.

15. Vorrichtung nach einem der Ansprüche 1 bis 14, **dadurch gekennzeichnet, dass** die Defibrillationselektrode (4) und die von ihr aufgenommene Stimulationselektrode (5) in zusammengesteckter Lage implantierbar sind, wobei die Defibrillationselektrode (4) relativ zu der Stimulationselektrode (5) soweit verschoben ist, dass sie das Verankerungsende oder eine dieses bildende, Schraubwendel (20) der Stimulationselektrode (5) in sich aufnimmt und umschließt, und dass am Ende des Implantationsvorgangs die Defibrillationselektrode (4) relativ zu der Stimulationselektrode (5) in ihre Gebrauchsstellung zurückziehbar und justierbar und/oder festlegbar ist.

## Claims

1. Device (1) for defibrillating a heart (2), having an implantable cardiac pacemaker and defibrillator (3), having at least one defibrillating electrode (4) and associated counter-electrode therefor, and having at least one stimulating electrode (5) which is also implantable, the defibrillating electrode (4) being connected to the stimulating electrode (5) in the position of use such that the pole (6) of the defibrillating electrode (4) is arranged on the outside of the stimulating electrode (5), **characterised in that** the defibrillating electrode is movable relative to the stimulating electrode (5) and can be retracted from its position of use relative to the implantable stimulating electrode (5) and replaced.

2. Device according to claim 1, **characterised in that** the pole (6) of the defibrillating electrode (4) is embodied as an electrically conductive coil which is arranged on a holder that is movable relative to the stimulating electrode (5), and which can be retracted with the coil that forms the defibrillating electrode (4) or its pole (6) and replaced.

3. Device according to claim 1 or 2, **characterised in that** the holder for the defibrillating electrode is an internal tube (8) which is movably mounted on the stimulating electrode (5) in the position of use.

4. Device according to one of claims 1 to 3, **characterised in that** the stimulating electrode (5) comprises a releasable coupling or separating point (10) in its path close to the cardiac pacemaker (3) or its plug (9) or is releasably connected to the cardiac pacemaker (3), and **in that** the holder or the tube (8) with the defibrillating electrode (4) and its connection or connections (11) as plug portion is provided with a branch (12) for the connections (11), wherein said plug portion can be pushed onto the part of the stimulating electrode (5) leading to the heart (2) after the release of the stimulating electrode (5) from the cardiac pacemaker (3) or at the separating point (10) and can be moved thereon or retracted therefrom.

5. Device according to one of claims 1 to 4, **characterised in that** the holder that comprises the defibrillating electrode (4) or the pole (6) thereof is embodied to be movable and securable on the stimulating electrode (5) with the branch (12) or parallel holding device and a fixing point.

6. Device according to one of claims 1 to 5, **characterised in that** the holder or the tube (8) comprises, adjacent to the branch (12), a clip (13) which is movable relative to the stimulating electrode (5) in the released position and is fixed thereto in the clamped position.

7. Device according to one of claims 1 to 6, **characterised in that** the clip (13) for fixing the defibrillating electrode (4) on the stimulating electrode (5) comprises a sleeve with an inner cavity extending axially through it to accommodate the stimulating electrode (5), the sleeve containing an insert that is elastically deformable in the axial direction counter to a restoring force or an expandable and/or elastic tube which simultaneously forms the longitudinal inner cavity for accommodating the stimulating electrode (5), and **in that** the sleeve is subdivided in the longitudinal direction, along a separating point (14) running around its circumference, into at least two parts (15, 16) that are movable relative to one another in the axial direction, the circumferential separating point (14) deviating axially on opposite sides alternately with respect to a diametric plane or being profiled or serrated, so that the edges of the parts (15, 16) of the sleeve that make contact at their end faces are interlockingly engaged in the direction of rotation, and **in that** the parts (15, 16) can be pulled apart in the axial direction counter to the restoring force of the elastic insert or tube that holds them together, to such an extent that the mutual profiling is released and the two parts (15, 16) can be rotated relative to each other by twisting or distorting the elastic insert or tube and can once more be secured in the rotated position in contact with one another to prevent any rotational movements.

8. Device according to one of claims 1 to 7, **characterised in that** the coil that forms the defibrillation pole (6) is subdivided in the longitudinal direction into at least two parts the spacing of which is selected so that one part is intended to be located in the ventricle and the other part is intended to be located in the atrium of the heart (2).

9. Device according to one of claims 1 to 8, **characterised in that** the spacing of the parts that form the defibrillation pole (6) is variable.

10. Device according to one of claims 1 to 9, **characterised in that** in order to vary the distance of the parts that form the defibrillation pole (6) these parts are arranged on holders or tube sections arranged concentrically with one another, the longer holder or the longer tube section of which carries the part of the defibrillating electrode (4) that is located in the ventricle in the position of use, and **in that** the other part of the coil of the defibrillating electrode (4) that is adjustable or movable relative thereto is arranged on a shorter holder or tube section that is disposed on the outside of the first-mentioned holder or tube section.

11. Device according to one of claims 1 to 10, **characterised in that** in the position of use the two tube sections are fixedly connected or attached by means of a clip (13) and when the defibrillating electrode is replaced they can be moved or withdrawn jointly relative to the stimulating electrode (5).

12. Device according to one of claims 1 to 11, **characterised in that** the pole region of the defibrillating electrode (4), in particular the coil of the defibrillating electrode (4) that forms the pole or poles (6) is covered with an insulating or silicone tubing (18) having individual openings (17) or ports.

13. Device according to one of claims 1 to 12, **characterised in that** the openings (17) or ports in the insulating tubing (18) are slots or perforations or at least one interruption extending over at least part of the circumference of the insulating tubing (18).

14. Device according to one of claims 1 to 13, **characterised in that** at least one platinum sleeve (19) is arranged in the region of an opening, a port or an interruption in the insulating tubing (18) on the coil that forms the defibrillating electrode.

15. Device according to one of claims 1 to 14, **characterised in that** the defibrillating electrode (4) and the stimulation electrode (5) which it accommodates are implantable in the assembled position, the defibrillating electrode (4) having been advanced relative to the stimulation electrode (5) such that it accommodates and surrounds the anchoring end or a helical coil (20) of the stimulating electrode that forms it, and **in that** at the end of the implanting process, in its position of use, the defibrillating electrode (4) is adapted to be withdrawn and adjusted and/or secured relative to the stimulating electrode.

## Revendications

1. Dispositif (1) de défibrillation d'un coeur (2) avec un stimulateur cardiaque implantable et un défibrillateur (3), avec au moins une électrode de défibrillation (4) et une contre-électrode associée à celle-ci ainsi qu'avec au moins une électrode de stimulation (5) également implantable, l'électrode de défibrillation (4) étant reliée, en position d'utilisation, à l'électrode de stimulation (5) de telle manière que le pôle (6) de l'électrode de défibrillation (4) soit disposé sur le côté extérieur de l'électrode de stimulation (5), **caractérisé en ce que** l'électrode de défibrillation peut coulisser par rapport à l'électrode de stimulation (5) et, par rapport à l'électrode de stimulation (5) implantée, être retirée de sa position d'utilisation et remplacée.

2. Dispositif selon la revendication 1, **caractérisé en ce que** le pôle (6) de l'électrode de défibrillation (4) est réalisé sous la forme d'une spirale électroconductrice qui est disposée sur un support coulissant par rapport à l'électrode de stimulation (5), lequel peut être retiré avec la spirale formant l'électrode de défibrillation (4) ou son pôle (6) et remplacé.

3. Dispositif selon la revendication 1 ou 2, **caractérisé en ce que** le support pour l'électrode de défibrillation est un tuyau intérieur (8) qui est disposé de manière coulissante sur l'électrode de stimulation (5) en position d'utilisation.

4. Dispositif selon une des revendications 1 à 3, **caractérisé en ce que** l'électrode de stimulation (5) présente sur son parcours à proximité du stimulateur cardiaque (3) ou de son connecteur (9) un accouplement détachable ou point de séparation (10) ou est reliée de manière détachable au stimulateur cardiaque (3) et que le support ou le tuyau (8) avec l'électrode de défibrillation (4) et son raccord ou ses raccords (11) est muni, en tant que partie enfichable, d'une dérivation (12) pour les raccords (11), laquelle partie enfichable, après la désolidarisation de l'électrode de stimulation (5) du stimulateur cardiaque (3) ou au point de séparation (10), est enfichable sur la partie de l'électrode de stimulation (5) conduisant au coeur (2) et peut coulisser sur celle-ci ou être retirée de celle-ci.

5. Dispositif selon une des revendications 1 à 4, **caractérisé en ce que** le support présentant l'électrode de défibrillation (4) ou son pôle (6) avec la dérivation (12) ou un dispositif de retenue parallèle et un point de fixation est réalisé de manière à pouvoir coulisser et être fixé sur l'électrode de stimulation (5).

6. Dispositif selon une des revendications 1 à 5, **caractérisé en ce que** le support ou le tuyau (8) présente au voisinage de la dérivation (12) une pince (13) qui, en position desserrée, peut coulisser par rapport à l'électrode de stimulation (5) et, en position serrée, est fixée sur celle-ci.

7. Dispositif selon une des revendications 1 à 6, **caractérisé en ce que** la pince (13) servant à fixer l'électrode de défibrillation (4) sur l'électrode de stimulation (5) présente une douille avec une cavité intérieure traversante en direction axiale pour recevoir l'électrode de stimulation (5), la douille contenant un insert déformable élastiquement en direction axiale contre une force de rappel ou un tuyau extensible et/ou élastique qui forme ou comprend en même temps la cavité intérieure longitudinale destinée à recevoir l'électrode de stimulation (5), et que la douille est divisée en direction longitudinale, le long d'un point de séparation (14) s'étendant sur son pourtour, en au moins deux parties (15, 16) mobiles l'une par rapport à l'autre en direction axiale, le point de séparation (14) s'étendant en s'écartant alternativement vers des côtés opposés axialement par rapport à un plan diamétral ou est profilé ou denté, de façon que les bords des pièces (15, 16) de la douille qui sont en contact frontal soient reliés par complémentarité de formes en direction de rotation, et que les pièces (15, 16) peuvent être séparées l'une de l'autre en direction axiale contre la force de rappel de l'insert ou du tuyau élastique qui les maintient ensemble jusqu'à ce que le profilage mutuel soit desserré et que les deux pièces (15, 16) puissent tourner l'une par rapport à l'autre par déformation ou torsion de l'insert ou du tuyau élastique et, dans la position tournée, être remises en contact mutuel et fixées de manière à empêcher tout mouvement de rotation.

8. Dispositif selon une des revendications 1 à 7, **caractérisé en ce que** la spirale formant le pôle de défibrillation (6) est divisée en direction longitudinale en au moins deux sections dont la distance est choisie de façon qu'une section soit prévue pour être disposée dans le ventricule et l'autre section pour être disposée dans l'oreillette du coeur (2).

9. Dispositif selon une des revendications 1 à 8, **caractérisé en ce que** la distance des sections formant le pôle de défibrillation (6) est modifiable.

10. Dispositif selon une des revendications 1 à 9, **caractérisé en ce que** pour modifier la distance des sections formant le pôle de défibrillation (6), celles-ci sont disposées respectivement sur des supports ou morceaux de tuyau disposés concentriquement l'un à l'autre, dont le support plus long ou le morceau de tuyau plus long porte la section de l'électrode de défibrillation (4) qui, en position d'utilisation, est disposée dans le ventricule, et que l'autre section de la spirale de l'électrode de défibrillation (4), déplaçable ou coulissante par rapport à la première section, est disposée sur un support plus court, disposé sur le coté extérieur du support nommé en premier ou du morceau de tuyau ou sur un morceau de tuyau.

11. Dispositif selon une des revendications 1 à 10, **caractérisé en ce que** les deux morceaux de tuyau sont reliés rigidement ou par une pince (13) en position d'utilisation et, lors du remplacement de l'électrode de défibrillation, peuvent coulisser ou être retirés ensemble par rapport à l'électrode de stimulation (5).

12. Dispositif selon une des revendications 1 à 11, **caractérisé en ce que** la zone polaire de l'électrode de défibrillation (4), en particulier la spirale formant le ou les piles (6) de l'électrode de défibrillation, est recouverte d'un tuyau isolant ou en silicone (18) présentant un certain nombre d'ouvertures (17) ou fenêtres.

13. Dispositif selon une des revendications 1 à 12, **caractérisé en ce que** les ouvertures (17) ou fenêtres du tuyau isolant (18) sont des fentes ou des trous ou au moins une interruption s'étendant sur au moins une partie du pourtour du tuyau isolant (18).

14. Dispositif selon une des revendications 1 à 13, **caractérisé en ce qu'**au niveau d'une ouverture, d'une fenêtre ou d'une interruption du tuyau isolant (18), au moins une douille en platine (19) est disposée sur la spirale formant l'électrode de défibrillation.

15. Dispositif selon une des revendications 1 à 14, **caractérisé en ce que** l'électrode de défibrillation (4) et l'électrode de stimulation (5) portée par celle-ci sont implantables dans l'état assemblé par emboîtement, l'électrode de défibrillation (4) étant déplacée par rapport à l'électrode de stimulation (5) jusqu'à ce qu'elle reçoive en elle et entoure l'extrémité d'ancrage ou une spirale de fixation (20) formant celle-ci de l'électrode de stimulation (5), et qu'à la fin de l'opération d'implantation l'électrode de défibrillation (4) peut être retirée et réglée et/ou fixée par rapport à l'électrode de stimulation (5) dans sa position d'utilisation.
